# FASCICULE DE BREVET EUROPEEN

(11) **EP 2 571 848 B1**
(45) Date de publication et mention de la délivrance du brevet: **23.07.2014**
(21) Numéro de dépôt: 11721751.3
(22) Date de dépôt: 17.05.2011
(51) Int. Cl.: C07C 253/00

(54) **PROCEDE DE FABRICATION DE COMPOSES COMPRENANT DES FONCTIONS NITRILES**
VERFAHREN ZUR HERSTELLUNG VON VERBINDUNGEN MIT NITRILFUNKTIONEN
PROCESS FOR PRODUCING COMPOUNDS COMPRISING NITRILE FUNCTIONS

(30) Priorité: 21.05.2010 FR 1053967
(43) Date de publication de la demande: 27.03.2013
(73) Titulaire: Rhodia Operations, 93306 Aubervilliers (FR)
(72) Inventeur: JACQUOT, Roland, F-69340 Francheville (FR); MARION, Philippe, F-69390 Vernaison (FR)
(74) Mandataire: Chatelan, Florence Anne
(86) Numéro de dépôt international: PCT/EP2011/057981
(87) Numéro de publication internationale: WO 2011/144619

(56) Documents cités:
- US-A- 2 444 828
- LAECKMANN D ET AL: "Synthesis and biological evaluation of aroylguanidines related to amiloride as inhibitors of the human platelet Na<+>/H<+> exchanger", BIOORGANIC & MEDICINAL CHEMISTRY, PERGAMON, GB LNKD- DOI:10.1016/S0968-0896(02)00022-6, vol. 10, no. 6, 1 janvier 2002 (2002-01-01), pages 1793-1804, XP002429666, ISSN: 0968-0896
- R. SELDNER: "Glutarimide", AMERICAN CHEMICAL JOURNAL, vol. 17, 1895, pages 532-535, XP002601977,
- EDMUN H. MILLER: "Succinimid", JOURNAL OF THE AMERICAN CHEMICAL SOCIETY, vol. 16, 1894, pages 433-462, XP002601978,

## Description

La présente invention concerne la fabrication de composés comprenant des fonctions nitriles et de composés imide cycliques.

Elle se rapporte plus particulièrement à la fabrication de composés comprenant des fonctions nitriles à partir de composés comprenant des fonctions carboxyliques, avantageusement d'origine naturelle et renouvelable, et à partir d'un mélange N de dinitriles comprenant du méthyl-2 glutaronitrile (MGN), de l'éthyl-2 succinonitrile (ESN) et éventuellement de l'adiponitrile (AdN).

Les composés comprenant des fonctions nitriles sont des produits importants pour la fabrication de composés amines. Les composés dinitriles conduisent à des amines qui sont par exemple des monomères à l'origine de polymères tels que le polyamide, par exemple. Les composés mononitriles conduisent à des amines ou à des amides qui sont par exemple utilisés pour la fabrication de tensioactifs cationiques.

De nombreux procédés de synthèse de nitriles ont été proposés, notamment des procédés de synthèse à partir d'ammoniac et d'acides carboxyliques. Ces procédés utilisent principalement comme matière première de départ des composés hydrocarbures issus du raffinage du pétrole, et de l'ammoniac, qui est obtenu à partir d'hydrogène par des procédés de vaporeformage qui consomment beaucoup d'énergie.

Compte tenu de l'épuisement de la ressource pétrolière, de nombreux travaux de recherche sont entrepris pour développer des procédés de synthèse de ces composés, importants pour la fabrication de matériaux utilisés dans de nombreuses applications, à partir de matières premières ou ressources dites renouvelables, ou à partir de matières premières recyclées, qui sont habituellement détruites ou valorisées sous forme d'énergie. Généralement ces ressources renouvelables sont produites à partir de matière végétale cultivée ou non telle que les arbres, les plantes comme la canne à sucre, le maïs, le manioc, le blé, le colza, le tournesol, la palme, le ricin ou analogues, ou à partir de matière animale telle que les graisses (suif etc.).

Cette matière végétale ou animale est transformée par des procédés comprenant généralement plusieurs étapes mécaniques, chimiques et biologiques.

Par ailleurs, à propos des matières premières recyclées, la fabrication de l'adiponitrile, un grand intermédiaire chimique utilisé notamment dans la synthèse de l'hexaméthylène diamine et du caprolactame (monomères pour la fabrication des polyamides), obtenu par hydrocyanation du butadiène, génère un flux de sous-produits dinitriles comprenant majoritairement des composés dinitriles ramifiés comme le méthyl-2 glutaronitrile, l'éthyl-2 succinonitrile. Ce mélange de composés dinitriles ramifiés est obtenu par distillation pour le séparer de l'adiponitrile. Comme la séparation n'est généralement pas complète, le mélange de composés dinitriles ramifiés peut comprendre également une faible proportion d'adiponitrile.

Plusieurs solutions ont été proposées pour valoriser ces sous-produits ou mélanges. Une de celles-ci consiste à hydrogéner les composés dinitriles en amines primaires notamment pour produire de la méthyl-2 pentaméthylènediamine (MPMD), utilisée comme monomère pour la fabrication de polyamides particuliers ou comme intermédiaire pour la production de vitamine B3 (nicotinamide).
Ce procédé requiert des étapes de purification du méthyl-2 glutaronitrile et de la méthyl-2 pentaméthylènediamine.

Dans l'industrie, ces sous-produits sont également valorisés sous forme de vapeur ou d'énergie par combustion. Toutefois, cette combustion peut demander un traitement des gaz pour éliminer les oxydes d'azote produits et elle produit du gaz carbonique qui est rejeté dans l'atmosphère.

Le documentLAECKMANN Det al : « Synthesis and Bilological Evaluation of Aroylguanidines related to Amiloride as Inhibitros of the Human Platelet Na+/H+ Exchanger » décrit un procédé de synthèse de 3-méthyl-glutarimide à partir de 2-méthyl-glutaronitrile (MGN) par chauffage d'une solution dans l'acide acétique en présence d'acide sulfurique et d'eau.

Le document R. SELDNER « Glutarimide » décrit un procédé de synthèse d'un composé de glutarimide et d'acétonitrile par chauffage d'une solution de glutaronitrile dans l'acide acétique.

Le document EMUN H. MILLER « Succinimid » décrit un procédé de synthèse d'un composé de succinimide et d'acétonitrile par chauffage d'une solution de succinonitrile dans l'acide acétique.

Le document US 2,444,828 décrit un procédé de synthèse d'oléonitrile par chauffage d'un composé d'acide oléique et d'urée.

Il existe donc toujours un besoin et une demande importante de trouver de nouvelles voies de valorisation et transformation des composés dinitriles ramifiés comme le méthyl-2 glutaronitrile, l'éthyl-2 succinonitrile ou des mélanges en composés chimiques valorisables et économiquement intéressants.

A cet effet, l'invention propose un procédé de préparation d'au moins un nitrile de formule générale I

(NC)ᵥ-R₁-(CN)_{w}

et d'au moins les imides cycliques 3-méthyl-glutarimide et 3-éthyl-succinimide, par réaction entre au moins un acide carboxylique de formule générale II

(HOOC)ₓ-R₁-(COOH)_{y}

et un mélange N de dinitriles comprenant du méthyl-2 glutaronitrile (MGN), de l'éthyl-2 succinonitrile (ESN) et éventuellement de l'adiponitrile (AdN),
   avec
x, y est égal à 0 ou 1 avec (x+y) égal à 1 ou 2
v, w est égal à 0 ou 1 avec (v+w) égal à 1 ou 2
R₁ représente un groupement hydrocarboné linéaire ou ramifié, saturé ou insaturé, pouvant comprendre des hétéroatomes, comprenant :
   - de 4 à 34 atomes de carbone lorsque (x+y) est égal à 2,
   - de 2 à 22 atomes de carbone lorsque (x+y) est égal à 1

Avantageusement le mélange N de dinitriles est un mélange issu du procédé de fabrication de l'adiponitrile par double hydrocyanation du butadiène. Il correspond de préférence à la fraction de distillation permettant de séparer les dinitriles ramifiés (méthyl-2 glutaronitrile, éthyl-2 succinonitrile) de l'adiponitrile.

Ce mélange de dinitriles a généralement la composition pondérale suivante :
Méthyl-2 glutaronitrile : comprise entre 70 % et 95 %, de préférence entre 80 et 85%
Ethyl-2 succinonitrile : compris entre 5 % et 30 %, de préférence entre 8 et 12%
Adiponitrile : compris entre 0 % et 10 % , de préférence entre 1 et 5%
le complément à 100% correspondant à différentes impuretés

Le procédé de l'invention met en oeuvre un acide carboxylique de formule générale II telle que décrite ci-dessus.

Le radical R₁ peut être un radical aliphatique, un groupement comprenant un radical aromatique ou cycloaliphatique, il peut être fonctionnalisé par exemple par une fonction hydroxyle, une fonction ester etc.

Le composé de formule II peut par exemple être choisi parmi l'acide trichloroacétique, l'acide trifluoroacétique, l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linolénique et les dimères d'acides gras contenant 36 atomes de carbone, l'acide téréphtalique, l'acide isophtalique.

Dans le cadre du procédé de l'invention, on peut mettre en oeuvre un mélange d'acides carboxyliques de formule générale II. A titre d'exemple de mélange d'acides, on peut citer les cocoacides qui sont issus de l'huile de palme ou de l'huile de coco.

Avantageusement l'acide carboxylique de formule générale II est issu d'une matière renouvelable d'origine végétale ou animale.

Une matière ou ressource renouvelable est une ressource naturelle, animale ou végétale, dont le stock peut se reconstituer sur une période courte à l'échelle humaine. Il faut en particulier que ce stock puisse se renouveler aussi vite qu'il est consommé.

A la différence des matériaux issus de matières fossiles, les matières premières renouvelables contiennent une proportion importante de ¹⁴C. De préférence les nitriles de l'invention sont constitués de carbone organique issu de matières premières renouvelables. Ainsi cette caractéristique préférée pourrait être certifiée par détermination de la teneur en ¹⁴C selon l'une des méthodes décrites dans la norme ASTM D6866, notamment selon la méthode par spectrométrie de masse ou la méthode par spectrométrie à scintillation liquide qui sont décrites dans cette norme.

Ces ressources renouvelables sont généralement produites à partir de matière végétale cultivée ou non telle que les arbres, les plantes comme la canne à sucre, le maïs, le manioc, le blé, le colza, le tournesol la palme, le ricin ou analogues, ou à partir de matière animale telle que les graisses (suif etc.).

Par exemple, l'acide carboxylique de formule générale II peut être issu de ressources renouvelables telles que les huiles végétales ou les polysaccharides naturels tels que l'amidon ou la cellulose, l'amidon pouvant être extrait, par exemple, du maïs ou de la pomme de terre. Il peut en particulier provenir de divers procédés de transformation, notamment de procédés chimiques classiques, mais également de procédés de transformation par voie enzymatique ou encore par fermentation.

Lorsque le composé de formule II est un monoacide gras, ce dernier peut par exemple être obtenu à partir d'huile végétale ou animale par transformation chimique (hydrolyse des huiles).

Lorsque le composé de formule II est un diacide, ce dernier peut être obtenu par fermentation à partir d'un monoacide gras obtenu selon la méthode ci-dessus. Par exemple, il est possible d'utiliser la levure *Candida Tropicalis* modifiée afin de réaliser la conversion d'un monoacide en diacide. On pourra notamment se référer aux documents WO 91/06660 et US 4474882. Le diacide peut également être obtenu à partir d'huile végétale ou animale par transformation chimique.

Lorsque la matière première est un polysaccharide, le composé de formule II est généralement obtenu par fermentation

Avantageusement le composé de formule II est choisi parmi l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linoléniqu et les dimères d'acides gras contenant 36 atomes de carbone, l'acide téréphtalique.

L'acide azélaïque peut être obtenu à partir de l'acide oléïque, par ozonolyse.

L'acide heptanedioïque et l'acide sébacique peuvent être obtenus à partir d'huile de ricin.

L'acide dodécanedioïque peut être obtenu par bio-fermentation de l'acide dodécanoïque, également dénommé acide laurique, l'acide laurique pouvant être produit à partir d'huile de noix de coco ou d'huile de palme-kernel.

L'acide brassylique peut être obtenu à partir de l'acide érucique (notamment par ozonolyse), étant précisé que l'acide érucique se trouve sous forme d'ester dans le colza.

L'acide tetradécanedioïque peut être obtenu par bio-fermentation de l'acide myristique, l'acide myristique pouvant être produit à partir d'huile de noix de coco ou d'huile de palme-kernel.

L'acide hexadécanedioïque peut être obtenu par bio-fermentation de l'acide palmitique, ce dernier se trouvant dans l'huile de palme principalement.

L'acide octadécanedioïque peut être obtenu par bio-fermentation de l'acide stéarique, étant précisé que l'acide stéarique peut être présent dans toutes les huiles végétales mais surtout dans les graisses animales.

L'acide eicosanedioïque peut être obtenu par bio-fermentation de l'acide arachidique que l'on trouve majoritairement dans l'huile de colza.

L'acide docosanedioïque peut être obtenu par métathèse de l'acide undécylénique que l'on extrait de l'huile de ricin.

Le diacide linéaire aliphatique ayant 36 atomes de carbones est un dimère d'acide gras issu par exemple des sous-produits des résineux transformés par les procédés Kraft. Il peut également être obtenu par oligomérisation ou polymérisation d'acides gras monobasiques insaturés à longue chaîne hydrocarbonée (tels que l'acide linoléïque et l'acide oléïque), comme décrit notamment dans le document EP 0471566.

Le procédé de l'invention est avantageusement mis en oeuvre à une température comprise entre 150 et 350°C. La pression mise en oeuvre est généralement comprise entre la pression atmosphérique et quelques bars.

Des catalyseurs peuvent être mis en oeuvre dans le cadre du procédé de l'invention. A titre d'exemple de catalyseurs, on peut citer l'acide phosphorique, les phosphates, les borophosphates, l'acide sulfurique, l'acide sulfonique, les acide benzène sulfonique, les acide toluène sulfonique tels que l'acide para toluène sulfonique, les acides naphtalène sulfoniques, la silice, l'alumine, l'argile, la silice/alumine.

De façon avantageuse, on met en oeuvre une quantité de mélange N de telle sorte qu'au moins une molécule de méthyl-2 glutaronitrile (MGN) ou d'éthyl-2 succinonitrile (ESN) est introduite dans le milieu réactionnel, par fonction acide de l'acide carboxylique de formule générale II à transformer en fonction nitrile.

Lorsque l'on met en oeuvre un diacide comme acide de formule générale II, on peut obtenir le dinitrile correspondant ou le nitrile acide correspondant (par exemple en mettant en oeuvre un défaut de fonction nitrile).

Lors de la réaction entre le composé de formule (II) et le mélange N de dinitriles conformément à l'invention, des imides sont formés, notamment le 3-méthyl glutarimide issu du MGN et le 3-éthylsuccinimide issu de l'ESN.

Avantageusement, le procédé de l'invention comprend également une étape de récupération d'au moins le nitrile de formule (I) d'une part, et d'au moins l'imide cyclique d'autre part, à partir du milieu réactionnel.

Cette récupération peut être réalisée par séparation des composés du milieu réactionnel, selon toute méthode connue telle que la distillation.

Selon un premier mode de réalisation avantageux, les composés peuvent être obtenus par distillation réactive. En effet lorsque le nitrile de formule (I) que l'on souhaite obtenir a une température d'ébullition inférieure à celle de la température de réaction (ce qui est en particulier le cas pour les nitriles ayant un faible nombre de carbones), on peut distiller ce nitrile au fur et à mesure de sa formation, ce qui déplace l'équilibre de la réaction en faveur de la formation de ce nitrile ; ceci est donc particulièrement avantageux. Cette méthode de distillation réactive peut par exemple être utilisée lorsque le nitrile de formule (I) est l'octanitrile ou le nonanitrile.

Selon un deuxième mode de réalisation avantageux, les composés peuvent être séparés par extraction à l'eau chaude. En effet les imides sont généralement solubles dans l'eau, contrairement notamment aux nitriles gras, ce qui permet une bonne séparation par une voie aisée à mettre en oeuvre. Cette voie est à privilégier notamment lorsque les nitriles et les imides à séparer ont des températures d'ébullition qui sont proches et qu'ils sont par ce fait difficile à séparer par distillation classique par exemple. Cette méthode d'extraction à l'eau chaude peut par exemple être utilisée lorsque le nitrile de formule (I) est le lauronitrile, l'oléonitrile ou lorsque l'on met en oeuvre un mélange d'acides de formule (II). La température de l'eau lors de cette extraction est généralement supérieure ou égale à 50°C.

Selon un mode de réalisation particulier de l'invention, le nitrile de formule (I) ainsi récupéré est hydrogéné pour former l'amine correspondante, selon une méthode connue de l'homme du métier. Ainsi on obtient une amine dont tous les carbones sont biosourcés (car issus d'un acide carboxylique biosourcé , c'est-à-dire un acide carboxylique issu d'une matière première renouvelable), et dont les atomes d'azote sont recyclés (car issus de sous-produits habituellement brûlés ce qui génère du dioxyde de carbone et des oxydes d'azote, gaz à effet de serre qu'il faut traiter pour satisfaire la législation en vigueur). De telles amines peuvent être utilisées comme matières premières pour la fabrication de polyamide, qui seront ainsi partiellement ou complètement biosourcés en fonction des acides utilisés pour la polymérisation. Ces amines peuvent également être utilisées pour préparer des tensioactifs.

Selon un autre mode de réalisation particulier de l'invention, on peut faire réagir l'imide cyclique récupéré selon le procédé de l'invention, avec un alcool pour former le diester correspondant. Un tel procédé est connu et notamment décrit dans le document WO2008/009792 et WO 2009/056477. Les diesters peuvent être utilisés comme solvants.

D'autres détails ou avantages de l'invention apparaîtront plus clairement à la vue des exemples donnés ci-dessous.

### EXEMPLES

### Exemples 1 à 4 : Préparation de dinitriles

### Exemple 1 : Nitrilation de l'acide sébacique

Dans un réacteur de 2 litres agité par un agitateur mécanique et équipé d'un réfrigérant à reflux , on introduit sous agitation 930 g (8,6 mole) d'un mélange de dinitriles (85% MGN, 12 % ESN, 3% AdN en poids) sous-produits de la synthèse de l'adiponitrile, puis on ajoute 8,5 g d'acide orthophosphorique à 85%. Toujours sous agitation, on introduit 809 g (4 mole) d'acide sébacique biosourcé. Le milieu réactionnel est ensuite chauffé progressivement au reflux c'est-à-dire à environ 285°C. Ces conditions sont maintenues pendant 4 heures puis le mélange réactionnel est refroidi à température ambiante. Ensuite, on distille successivement le mélange de 3-méthylglutarimide, 3-éthylsuccinimide et 2-méthylglutaronitrile mis en excès vers 140°C sous 8 mmHg et on termine par la distillation du sébaconitrile à 185°C sous 12 mmHg.

Les rendements respectifs en mélange d'imides et de sébaconitrile sont de 95% et 92%. Dans une seconde étape, les imides sont traités par un alcool et notamment le méthanol pour obtenir un mélange de diesters méthyliques (2-méthyl glutarate de méthyle et 2-éthylsuccinate de méthyle). Le sébaconitrile est hydrogéné en diamine correspondante selon le mode opératoire décrit à l'exemple 2.

### Exemple 2 : Hydrogénation du sébaconitrile

Dans un autoclave de 750 ml équipé d'une turbine auto-aspirante, on introduit 50 g de 1,12 décanediamine et 50g d'eau. On ajoute 10 g de nickel de Raney dopé par 2% en poids de Cr et basifié à la soude. Le réacteur est purgé à l'azote, puis à l'hydrogène 2 fois avec 10 bars de pression. On chauffe sous pression de 20 bars d'hydrogène à 90°C. On introduit alors avec une pompe le sébaconitrile de l'exemple 1, on injecte en 4 heures 400g de dinitrile. La réaction est exothermique. La température est maintenue constante par refroidissement. Après la fin de l'injection, on laisse 15 min à 90°C sous 20 bars d'hydrogène. On ramène à température ambiante et on purge le réacteur à l'azote. Le catalyseur est filtré et partiellement recyclé. On admet une désactivation de catalyseur de 1g de Nickel pour 1kg de dinitrile hydrogéné. La diamine est ensuite purifiée par distillation sous pression réduite. On récupère la diamine à 140°C sous 10mmHg, celle-ci se solidifie à 62°C. Le rendement est de 95%. Pour avoir un rendement maximal de la réaction, il faut alimenter le nitrile à un débit suffisant sans accumuler le nitrile dans le milieu réactionnel.

### Exemple 3 : Nitrilation de l'acide téréphtalique

On introduit dans un réacteur de 2000 ml, 650 g (6,0 mol) d'un mélange de dinitriles (85% MGN, 12 % ESN, 3% AdN en poids) sous produits de la synthèse de l'adiponitrile. Dans ce milieu hétérogène, on ajoute 167 g d'acide téréphtalique (1,0 mol) et ensuite on ajoute 5 g d'acide orthophosphorique à 85%. On chauffe le milieu réactionnel au reflux des dinitriles et on maintient dans ces conditions pendant 5 h. Par analyse CPG on obtient des rendements respectifs en mélange des imides et en 1,4- dicyanobenzène de 96 et 95%.

### Exemple 4 : Nitrilation de l'acide adipique

On introduit dans un réacteur de 500 ml 146,3 g d'acide adipique (1 mole) et 221 g (2,05 mole) d'un mélange de dinitriles (85% MGN, 12 % ESN, 3% AdN en poids) sous produits de la synthèse de l'adiponitrile. On ajoute à la suspension jaunâtre 2,0 g d'acide orthophosphorique à 85% et on chauffe le milieu en agitant au reflux. On maintient dans ces conditions pendant 3 heures. Par analyse CPG, on obtient les résultats suivants
94% de conversion du mélange de dinitriles
95% de rendement en mélange d'imides
75% de rendement en adiponitrile

### Exemples 5 à 11 : Préparation de mononitriles

### Exemple 5 : Nitrilation de l'acide pélargonique

Dans un réacteur de 2 litres équipé d'une agitation mécanique et d'un réfrigérant à reflux, on introduit 930 g (8,6 mole) d'un mélange de dinitriles (85% MGN, 12 % ESN, 3% AdN en poids) sous produits de la synthèse de l'adiponitrile. On ajoute 8,0 g d'acide orthophosphorique à 85%. On agite et on introduit 1265g (8,0 mole) d'acide pélargonique biosourcé. On chauffe progressivement le milieu réactionnel au reflux de l'acide pélargonique c'est-à-dire vers 269°C. On distille alors progressivement le nonanenitrile qui a un point d'ébullition inférieur (224°C) et par ce moyen on déplace l'équilibre de la réaction. Après 6 heures de chauffage, on récupère le nitrile avec un rendement de 95% par rapport à l'acide pélargonique engagé. On distille ensuite le MGN mis en excès et les imides qui peuvent être engagés dans la réaction d'estérification avec le méthanol.

### Exemple 6 : Hydrogénation du nonanenitrile

Dans un autoclave de 750 ml équipé d'une turbine auto-aspirante, on introduit 50g de nonylamine et 50g d'eau. On ajoute 5 g de nickel de Raney dopé par 2% en poids de Cr et basifié. On purge le réacteur avec 2 fois 10 bars d'azote puis par 2 fois 10 bars d'hydrogène. On place l'autoclave sous 20 bars d'hydrogène, on agite et on chauffe à 90°C. On injecte alors à l'aide d'une pompe le nonanenitrile issu de l'exemple 5 en maintenant la pression d'hydrogène constante dans le réacteur. On injecte en 4h 350g de nonanenitrile. Après la fin de l'injection, on maintient pendant 30min le milieu réactionnel dans les mêmes conditions. On ramène ensuite la température à 20°C, on purge le réacteur à l'azote et on filtre le catalyseur. Le milieu réactionnel est alors distillé sous pression réduite : on récupère de cette façon la nonylamine avec un rendement de 98%

### (point d'ébullition 201 °C sous 760 mmHg)

### Exemple 7 : Nitrilation de l'acide oléique

Dans un réacteur de 2000 mL équipé d'une agitation et d'un réfrigérant à reflux, on introduit 250 g (2,30 mol) d'un mélange de dinitriles (85% MGN, 12 % ESN, 3% AdN en poids) sous produits de la synthèse de l'adiponitrile , on ajoute ensuite sous agitation 564 g (2 mol) d'acide oléique biosourcé. On coule alors 5,0 g de catalyseur -l'acide orthophosphorique- à 85%. On chauffe toujours sous agitation au reflux du mélange des dinitriles à 270-275°C . On maintient dans ces conditions pendant 4 h. Par analyse CPG , on obtient une conversion de l'acide oléique de 98% et un rendement en somme des imides de 97% . Le rendement en oléonitrile étant de 94%. Le milieu réactionnel est alors lavé à 65°C avec 3 fois 250 g d'eau pour éliminer du milieu les imides formés. Par cette technique, on récupère l'oléonitrile avec une pureté de 97%.

### Exemple 8 : Nitrilation de l'acide oléique

On opère de la même façon qu'à l'exemple 7 mais sans ajouter de catalyseur. Dans ces conditions, le reflux est maintenu pendant 6h et on obtient une conversion de 97% de l'acide oléique et un rendement en somme des imides de 97%. Le rendement en oléonitrile est de 95%. Le milieu réactionnel peut également être traité avec de l'eau à 65°C comme dans l'exemple 7, pour séparer les imides et l'oléonitrile.

### Exemple 9 : Nitrilation de l'azélate de monométhyle

Dans un réacteur de 250 mL équipé d'une agitation mécanique, on introduit 40,5 g d'ester monométhylique de l'acide azélaique biosourcé et 21,6 g (0,2 mol) d'un mélange de dinitriles (85% MGN, 12 % ESN, 3% AdN en poids) sous produits de la synthèse de l'adiponitrile. On ajoute à la solution obtenue en agitant, 0,2 g d'acide orthophosphorique à 85%. On chauffe alors au reflux soit à environ 270°C. Après un maintien de 3 heures dans ces conditions, la conversion en ester monométhylique de l'acide azélaïque est totale et le rendement en mélange d'imines est de 91 %. Le rendement en cyanoester de l'acide azélaïque est de 85%.

### Exemple 10 : Mononitrilation de l'acide sébacique

Dans un réacteur de 500 mL équipé d'une agitation mécanique et chauffé par un chauffe ballon électrique, on introduit 202 g (1,0 mol) d'acide sébacique biosourcé et 108 g (1,0 mol) d'un mélange de dinitriles (85% MGN, 12 % ESN, 3% AdN en poids) sous produits de la synthèse de l'adiponitrile. On agite et on chauffe, lorsque le mélange est homogène, on introduit alors 0,4 g d'acide orthophosphorique à 85%. On porte la température du milieu au reflux c'est-à-dire à une température proche de 270°C. On maintient dans ces conditions pendant 4 heures. Après ce temps, on obtient un rendement de 65% en acide nitrile correspondant et un rendement de 90% en mélange d'imides correspondant au mélange de dinitriles.

### Exemple 11 : Nitrilation de l'acide laurique

Dans un réacteur de250 mL équipé d'une agitation mécanique et chauffé par un chauffe ballon électrique, on introduit 80 g (400 mmol) d'acide laurique biosourcé et 48 g (440 mmol) d'un mélange de dinitriles (85% MGN, 12 % ESN, 3% AdN en poids) sous produits de la synthèse de l'adiponitrile. On agite et on chauffe pour homogénéiser le milieu réactionnel et on ajoute 0,4 g d'acide orthophosphorique à 85%. On chauffe alors au reflux vers 270°C et on maintient dans ces conditions pendant 3 heures. On ramène alors la température du milieu vers 95°C et on ajoute 50 g d'eau. On agite pendant 15 min à 60°C et on décante pour récupérer les imides et partiellement les dinitriles mis en excès en solution aqueuse. La phase organique qui contient le lauronitrile est extraite encore 2 fois avec de nouveau 50 g d'eau toujours à 65°C. On récupère alors le lauronitrile avec un rendement de 92% et une pureté de 93%.

### Exemple 12 : Nitrilation de l'acide octanoïque

Dans un réacteur de 2000 mL équipé d'une agitation et d'un dispositif de distillation en continu on introduit 594 g (5,44 mol) d'un mélange de dinitriles (85% MGN, 12 % ESN, 3% AdN en poids) sous produits de la synthèse de l'adiponitrile. On ajoute 751 g (5,15 mol) d'acide octanoïque. Sous agitation, on introduit 1,3 g d'acide orthophosphorique à 85%. On chauffe le milieu réactionnel qui devient homogène vers 230 - 240°C. A cette température, l'octanitrile distille en continu dès sa formation. Après 5 heures de réaction, on obtient 55g d'octanitrile ce qui représente un rendement de 84%.

## Revendications

1. Procédé de préparation d'au moins un nitrile de formule générale I
(NC)ᵥ-R₁-(CN)_{w}
et d'au moins les imides cycliques 3-méthyl-glutarimide et 3-éthyl-succinimide,
par réaction entre au moins un acide carboxylique de formule générale II
(HOOC)ₓR₁-(COOH)_{y}
et un mélange N de dinitriles comprenant du méthyl-2 glutaronitrile (MGN), de l'éthyl-2 succinonitrile (ESN) et éventuellement de l'adiponitrile (AdN),
avec
x, y est égal à 0 ou 1 avec (x+y) égal à 1 ou 2
v, w est égal à 0 ou 1 avec (v+w) égal à 1 ou 2
R₁ représente un groupement hydrocarboné linéaire ou ramifié, saturé ou insaturé, pouvant comprendre des hétéroatomes, comprenant :
- de 4 à 34 atomes de carbone lorsque (x+y) est égal à 2,
- de 2 à 22 atomes de carbone lorsque (x+y) est égal à 1

2. Procédé selon la revendication 1, **caractérisé en ce que** le mélange N de dinitriles est un mélange issu du procédé de fabrication de l'adiponitrile par double hydrocyanation du butadiène.

3. Procédé selon la revendication 1 ou 2, **caractérisé en ce que** le mélange N de dinitriles a la composition pondérale suivante :
Méthyl-2 glutaronitrile : comprise entre 70 % et 95 %, de préférence entre 80 et 85%
Ethyl-2 succinonitrile : compris entre 5 % et 30 %, de préférence entre 8 et 12%
Adiponitrile : compris entre 0 % et 10 %, de préférence entre 1 et 5%
le complément à 100% correspondant à différentes impuretés

4. Procédé selon l'une des revendications précédentes, **caractérisé en ce que** le composé de formule Il est issu d'une matière renouvelable d'origine végétale ou animale

5. Procédé selon la revendication 4, **caractérisé en ce que** le composé de formule Il est choisi parmi l'acide caproïque, l'acide caprylique, l'acide pélargonique, l'acide caprique, l'acide laurique, l'acide myristique, l'acide palmitique, l'acide stéarique, l'acide arachidique, l'acide adipique, l'acide heptanedioïque, l'acide octanedioïque, l'acide azélaïque, l'acide sébacique, ou undécanedioïque, l'acide dodécanedioïque, l'acide brassylique, l'acide tetradécanedioïque, l'acide hexadécanedioïque, l'acide octadécanedioïque, l'acide eicosanedioïque, l'acide docosanedioïque, l'acide octadécènoïque, l'acide oléique, l'acide ricinoléique, l'acide érucique, l'acide linoléique, l'acide linolénique et les dimères d'acides gras contenant 36 atomes de carbone, l'acide téréphtalique.

6. Procédé selon l'une des revendications précédentes, **caractérisé en ce qu'**il comprend une étape de récupération d'au moins le nitrile d'une part, et d'au moins les imides cycliques 3-méthyl-glutarimide et 3-éthyl-succinimide d'autre part, par séparation des composés du milieu réactionnel

7. Procédé selon la revendication 6, **caractérisé en ce qu'**au moins le nitrile récupéré est hydrogéné pour former l'amine correspondante.

8. Procédé selon la revendication 6 ou 7, **caractérisé en ce que** l'on fait réagir au moins les imides cycliques 3-méthyl-glutarimide et 3-éthyl-succinimide récupérés avec un alcool pour former le diester correspondant

## Patentansprüche

1. Verfahren zur Herstellung mindestens eines Nitrils der allgemeinen Formel I
(NC)ᵥ-R₁- (CN)_{w}
und mindestens der cyclischen Imide 3-Methylglutarimid und 3-Ethylsuccinimid,
durch Reaktion zwischen mindestens einer Carbonsäure der allgemeinen Formel II
(HOOC)ₓ-R₁-(COOH)_{y}
und einem Gemisch N von Dinitrilen, die 2-Methylglutaronitril (MGN), 2-Ethylsuccinonitril (ESN) und gegebenenfalls Adiponitril (AdN) umfasst,
wobei
x und y gleich 0 oder 1 sind, wobei (x+y) gleich 1 oder 2 ist,
v und w gleich 0 oder 1 sind, wobei (v+w) gleich 1 oder 2 ist,
R₁ für eine gesättigte oder ungesättigte, lineare oder verzweigte Kohlenwasserstoffgruppe, die Heteroatome umfassen kann, mit:
- 4 bis 34 Kohlenstoffatomen, wenn (x+y) gleich 2 ist,
- 2 bis 22 Kohlenstoffatomen, wenn (x+y) gleich 1 ist,
steht.

2. Verfahren nach Anspruch 1, **dadurch gekennzeichnet, dass** es sich bei dem Gemisch N von Dinitrilen um ein Gemisch aus dem Verfahren zur Herstellung von Adiponitril durch doppelte Hydrocyanierung von Butadien handelt.

3. Verfahren nach Anspruch 1 oder 2, **dadurch gekennzeichnet, dass** das Gemisch N von Dinitrilen die folgende gewichtsbezogene Zusammensetzung aufweist:
Methyl-2-glutaronitril: zwischen 70% und 95%, vorzugsweise zwischen 80 und 85%
Ethyl-2-succinonitril: zwischen 5% und 30%, vorzugsweise zwischen 8 und 12%
Adiponitril: zwischen 0% und 10%, vorzugsweise zwischen 1 und 5%,
wobei der Rest auf 100% verschiedenen Verunreinigungen entspricht.

4. Verfahren nach einem der vorhergehenden Ansprüche, **dadurch gekennzeichnet, dass** die Verbindung der Formel II aus einem erneuerbaren Stoff pflanzlichen oder tierischen Ursprungs stammt.

5. Verfahren nach Anspruch 4, **dadurch gekennzeichnet, dass** die Verbindung der Formel II aus Capronsäure, Caprylsäure, Pelargonsäure, Caprinsäure, Laurinsäure, Myristinsäure, Palmitinsäure, Stearinsäure, Arachidinsäure, Adipinsäure, Heptandisäure, Octandisäure, Azelainsäure, Sebacinsäure, oder Undecandisäure, Dodecandisäure, Brassylsäure, Tetradecandisäure, Hexadecandisäure, Octadecandisäure, Eicosandisäure, Docosandisäure, Octadecensäure, Ölsäure, Ricinolsäure, Erucasäure, Linolsäure, Linolensäure und Fettsäuredimeren mit 36 Kohlenstoffatomen und Terephthalsäure ausgewählt wird.

6. Verfahren nach einem der vorhergehenden Ansprüche, **dadurch gekennzeichnet, dass** es einen Schritt der Gewinnung mindestens des Nitrils einerseits und mindestens der cyclischen Imide 3-Methylglutarimid und 3-Ethylsuccinimid andererseits durch Abtrennung der Verbindungen aus dem Reaktionsmedium umfasst.

7. Verfahren nach Anspruch 6, **dadurch gekennzeichnet, dass** mindestens ein Teil des gewonnenen Nitrils zu dem entsprechenden Amin hydriert wird.

8. Verfahren nach Anspruch 6 oder 7, **dadurch gekennzeichnet, dass** man mindestens die gewonnenen cyclischen Imide 3-Methylglutarimid und 3-Ethylsuccinimid mit einem Alkohol zu dem entsprechenden Diester umsetzt.

## Claims

1. Process for preparing at least one nitrile of general formula I
(NC)ᵥ-R₁-(CN)_{w}
and at least the cyclic imides 3-methylglutarimide and 3-ethylsuccinimide,
by reaction between at least one carboxylic acid of general formula II
(HOOC)ₓ-R₁-(COOH)_{y}
and a mixture N of dinitriles comprising 2-methylglutaronitrile (MGN), 2-ethylsuccinonitrile (ESN) and optionally adiponitrile (AdN),
with
x, y is equal to 0 or 1 with (x+y) equal to 1 or 2
v, w is equal to 0 or 1 with (v+w) equal to 1 or 2
R₁ represents a linear or branched, saturated or unsaturated hydrocarbon-based group which may comprise heteroatoms, comprising:
- from 4 to 34 carbon atoms when (x+y) is equal to 2,
- from 2 to 22 carbon atoms when (x+y) is equal to 1.

2. Process according to Claim 1, **characterized in that** the mixture N of dinitriles is a mixture resulting from the process for producing adiponitrile by double hydrocyanation of butadiene.

3. Process according to Claim 1 or 2, **characterized in that** the mixture N of dinitriles has the following composition by weight:
2-Methylglutaronitrile: between 70% and 95%, preferably between 80% and 85%,
2-Ethylsuccinonitrile: between 5% and 30%, preferably between 8% and 12%,
Adiponitrile: between 0% and 10%, preferably between 1% and 5%,
the rest to 100% corresponding to various impurities.

4. Process according to one of the preceding claims, **characterized in that** the compound of formula II is derived from a renewable matter of vegetable or animal origin.

5. Process according to Claim 4, **characterized in that** the compound of formula II is chosen from caproic acid, caprylic acid, pelargonic acid, capric acid, lauric acid, myristic acid, palmitic acid, stearic acid, arachidic acid, adipic acid, heptanedioic acid, octanedioic acid, azelaic acid, sebacic acid, or undecanedioic acid, dodecanedioic acid, brassylic acid, tetradecanedioic acid, hexadecanedioic acid, octadecanedioic acid, eicosanedioic acid, docosanedioic acid, octadecenoic acid, oleic acid, ricinoleic acid, erucic acid, linoleic acid, linolenic acid and fatty acid dimers containing 36 carbon atoms, and terephthalic acid.

6. Process according to one of the preceding claims, **characterized in that** it comprises a step of recovering, on the one hand, at least the nitrile and, on the other hand, at least the cyclic imides 3-methylglutarimide and 3-ethylsuccinimide, by separation of the compounds from the reaction medium.

7. Process according to Claim 6, **characterized in that** at least the recovered nitrile is hydrogenated so as to form the corresponding amine.

8. Process according to Claim 6 or 7, **characterized in that** at least the recovered cyclic imides 3-methylglutarimide and 3-ethylsuccinimide are reacted with an alcohol so as to form the corresponding diester.
